# EUROPÄISCHE PATENTANMELDUNG

(11) **EP 1 457 232 A1**
(43) Veröffentlichungstag der Anmeldung: **15.09.2004**
(21) Anmeldenummer: 03450064.5
(22) Anmeldetag: 11.03.2003
(51) Int. Cl.: A61N 1/16

(54) **Anlage zum Entstören von geopathologischen Zonen**

(71) Anmelder: Kerschbaumer, Kuno, 8820 Neumarkt (AT); Kerschbaumer, Irene, 8820 Neumarkt (AT)
(72) Erfinder: Kerschbaumer, Kuno, 8820 Neumarkt (AT); Kerschbaumer, Irene, 8820 Neumarkt (AT)
(74) Vertreter: Ellmeyer, Wolfgang

(57) **Zusammenfassung**

Anlage zum Entstören von geopathologischen Zonen, wobei eine erste und eine zweite Boden-Einbringungselektrode (7, 10), eine erste und eine zweite Boden-Ausgangselektrode (8, 9) und eine Gleichrichterschaltung (1) mit zwei Wechselstrom-Eingängen (2, 3) und zwei Gleichstrom-Ausgängen (4, 5), von denen einer positiv und der andere negativ ist, vorgesehen sind, wobei die zwei Boden-Einbringungselektroden (7, 10) mit den zwei Wechselstrom-Eingängen (2, 3) und die zwei Boden-Ausgangselektroden (8, 9) mit den zwei Gleichstrom-Ausgängen (4, 5) verbunden sind, und im Betrieb die Gleichrichterschaltung (1) in einer ersten Tiefe (t1), die erste Boden-Einbringungselektrode (7) und die erste Boden-Ausgangselektrode (8) in einer zweiten Tiefe (t2), die geringer als die erste Tiefe (t1) ist, sowie die zweite Boden-Einbringungselektrode (10) und die zweite Boden-Ausgangselektrode (9) in einer dritten Tiefe (t3), die größer als die erste Tiefe (t1) ist, im Boden eingebracht sind.

## Beschreibung

Die Erfindung betrifft eine Anlage zum Entstören von geopathologischen Zonen.

Anlagen dieser Art dienen zum Entstören geopathologischer Interferenzen, zum Positivieren und Neutralisieren von allen auf der Erde erforschten und unerforschten geopathologischen Interferenzen, Verwerfungen, Störzonen, Wasseraderstrahlungen, Elektrosmog und Erdstrahlen mit ihren Gitternetzlinien und deren Kreuzungspunkten. Aus der Literatur bekannt sind bisher lediglich solche Anlagen, die mit aufbauendem Magnetismus arbeiten, die jedoch eine zusätzlich Belastung von Mensch und Tier durch den Magnetfeldaufbau mit positiver und negativer Strahlung darstellen.

Die Erfindung hat sich zum Ziel gesetzt, eine Anlage der eingangs erwähnten Art anzugeben, welche auf einen zusätzlichen Magnetfeldaufbau zur Gänze verzichtet, wobei die Mischspannung des Bodens, insbesondere des Erdreiches als unabhängiger, permanenter Energielieferant genutzt und damit ein dauerhafter Betrieb zur Entstörung, zum Positivieren und Neutralisieren von Störzonen innerhalb kurzer Zeit gewährleistet wird.

Erfindungsgemäß wird dies dadurch erreicht, daß eine erste und eine zweite Boden-Einbringungselektrode, eine erste und eine zweite Boden-Ausgangselektrode und eine Gleichrichterschaltung mit zwei Wechselstrom-Eingängen und zwei Gleichstrom-Ausgängen, von denen einer positiv und der andere negativ ist, vorgesehen sind, wobei die zwei Boden-Einbringungselektroden mit den zwei Wechselstrom-Eingängen und die zwei Boden-Ausgangselektroden mit den zwei Gleichstrom-Ausgängen verbunden sind, und daß im Betrieb die Gleichrichterschaltung in einer ersten Tiefe, die erste Boden-Einbringungselektrode und die erste Boden-Ausgangselektrode in einer zweiten Tiefe, die geringer als die erste Tiefe ist, sowie die zweite Boden-Einbringungselektrode und die zweite Boden-Ausgangselektrode in einer dritten Tiefe, die größer als die erste Tiefe ist, im Boden eingebracht sind.

Die im Boden vorherrschende Mischspannung wird der Gleichrichterschaltung über die Boden-Einbringungselektroden als Spannungspotential zugeführt und über die Boden-Ausgangselektroden gleichgerichtet in den Boden zurückgeleitet. Die von den Boden-Ausgangselektroden abgegebene Gleichspannung schafft ein neues gleichgerichtetes Spannungspotential im Boden. Auf diese Weise können geopathologische Interferenzen, Verwerfungen, Störzonen, Wasseraderstrahlungen, Elektrosmog und Erdstrahlen mit ihren Gitternetzlinien und Kreuzungspunkten, z.B. im Hartmannschen Netz, Benkerschen Kubensystem und im Curie-Netz samt deren Kreuzungspunkten ausgelöscht sowie die Rechtspolarisation von linksgedrehtem Quell- und Leitungswasser vorgenommen werden. Als bevorzugte zweite Tiefe, in der die erste Boden-Einbringungselektrode und die erste Boden-Ausgangselektrode eingebracht sind, hat sich 300 mm herausgestellt.

Eine erfindungsgemäß ausgestattete Anlage erreicht bereits nach 0,5 bis 2 Stunden die volle Leistungskapazität, und dann kann bereits ein entstörtes Erdreich, Gelände, Haus od. dgl. gemessen werden. Das bedeutet, daß die bekannten Gitternetze, wie z.B. das Hartmannsche Gitternetz, das Benkersche Kubensystem, das Curie-Gitternetz samt deren Kreuzungspunkten entstört werden und alle negativen Kreuzungen sich in positive Energie wandeln. Dasselbe gilt für alle pathogenen Störzonen, welche negative Energie aufweisen. In der Folge hat sich bei den Versuchen gezeigt, daß sich negativ (linksdrehendes) Wasser in dem mit der erfindungsgemäßen Anlage beeinflußten Gebiet immer in positives (rechtsdrehendes) Wasser gewandelt hat.

Bei Versuchen hat sich ferner gezeigt, daß sich nach Einbau der erfindungsgemäßen Anlage die zuvor durch unkompensierte Störzonen hervorgerufenen Erkrankungen, wie z.B. Migräne oder unerklärliche Schlafstörungen oder nicht nachvollziehbare Rückenschmerzen nicht mehr aufgetreten sind. Viele oft nicht erklärbare Beschwerden wurden innerhalb weniger Wochen reduziert oder gar eingedämmt.

Die erfindungsgemäße Anlage ist im Boden vorgesehen, so kann sie bevorzugt im Erdreich, in einem Mauerwerk oder in einem Fundament eingelassen sein. Es fallen aber auch andere Bodeneinbringungsformen unter den Schutzbereich der Erfindung.

Eine Gleichrichtung auch negativer Wellen der Boden-Mischspannung kann dann erfolgen, wenn die Gleichrichterschaltung durch einen Brückengleichrichter gebildet ist. Zur Vermeidung von Erdschlüssen zwischen den Anschlüssen der Gleichrichterschaltung ist in weiterer Ausbildung diese in einem elektrisch isolierenden Kunststoff-Gehäuse eingekapselt.

Die in den Boden über die Boden-Ausgangselektroden zurückgeführte Gleichspannung soll unter Umständen nur in bestimmten Richtungen im Boden zu einem Stromfluß führen. Daher können gemäß einem weiteren Ausführungsbeispiel der Erfindung die Boden-Ausgangselektroden teilweise mit einer elektrisch isolierenden Hülle umgeben sein.

Das Abgreifen der Mischspannung und das Wiedereinbringen dieser Spannung nach ihrer Gleichrichtung geschieht in bevorzugter Weise in gleichen Tiefenlagen des Bodens, weswegen die erste Boden-Einbringungselektrode und die erste Boden-Ausgangselektrode in Einbaulage im wesentlichen waagrecht und parallel zueinander angeordnet sind.

In bevorzugter Weise beträgt der Abstand der ersten Boden-Einbringungselektrode und der ersten Boden-Ausgangselektrode 300mm.

Eine weitere Ausführungsform kann darin bestehen, daß die zweite Boden-Einbringungselektrode und die zweite Boden-Ausgangselektrode in Einbaulage vertikal und parallel zueinander angeordnet sind. Auf diese Weise werden auch Mischspannungsfelder erfaßt, deren Maxima in vertikaler Richtung variieren.

Dabei hat es sich als vorteilhaft herausgestellt, wenn die Länge der zweiten Boden-Einbringungselektrode größer als die Länge der zweiten Boden-Ausgangselektrode ist. Es kann dabei bevorzugt die Länge der zweiten Boden-Einbringungselektrode ungefähr 1200 mm und die Länge der zweiten Boden-Ausgangselektrode ungefähr 800 mm betragen, wobei der Abstand zwischen der zweiten Boden-Einbringungselektrode und der zweiten Boden-Ausgangselektrode vorzugsweise 100 mm ist.

Um die Feldstärke der abgegebenen Gleichspannung innerhalb des Bodens in Richtung nach unten hin zu erhöhen, weisen die zweite Boden-Einbringungselektrode 10 und die zweite Boden-Ausgangselektrode 9 an ihren unteren Enden kegelförmige Spitzen auf, und die gegenüberliegenden oberen Enden sind stumpf ausgebildet und miteinander fluchtend angeordnet, um eine Referenzlinie gegenüber den waagrecht angeordneten, darüber in einem Abstand befindlichen ersten Boden-Einbringungs- und Ausgangselektroden zu definieren.

Das Abgreifen der Bodenmischspannung und die Rückführung des gleichgerichteten Potentials geschieht im wesentlichen in zwei voneinander beabstandeten Tiefenlagen. Eine vollständige Auslöschung geopathologischer Interferenzen läßt sich erzielen, wenn der Normalabstand zwischen den oberen Enden der zweiten Boden-Einbringungselektrode und der zweiten Boden-Ausgangselektrode einerseits und der ersten Boden-Einbringungselektrode und der ersten Boden-Ausgangselektrode andererseits 500 mm beträgt.

Eine in alle Richtungen gleichmäßige Wirkung läßt sich in Weiterbildung der Erfindung dadurch erreichen, daß die zweite Boden-Einbringungselektrode und die zweite Boden-Ausgangselektrode symmetrisch zu der vertikal zwischen der ersten Boden-Einbringungselektrode und der ersten Boden-Ausgangselektrode verlaufenden Mittelebene angeordnet sind.

Bei der Realisierung der erfindungsgemäßen Anlage ist darauf zu achten, daß es möglichst zu keinem störenden Magnetfeldaufbau kommt. Eine weitere Ausführungsform der Erfindung kann daher darin bestehen, daß die beiden Boden-Einbringungselektroden und die beiden Boden-Ausgangselektroden aus einem nicht-magnetischen Material, insbesondere Aluminium, gebildet sind.

Eine störungs- und verlustarme Zu- und Ableitung kann dann erreicht werden, wenn die Verbindungsleitungen zwischen der Gleichrichterschaltung und den Boden-Einbringungselektroden sowie den Boden-Ausgangselektroden durch isolierte Kupferleitungen gebildet sind, deren Querschnitt im Bereich von 0,15 mm² bis 1 mm² beträgt.

Ein weiteres Ziel der Erfindung besteht darin, die Qualität von Quell-, Grund- und Leitungswasser zu verbessern, indem Kalkablagerungen in Wasserzuleitungen und Wasser-Reservoirs verhindert werden.

Dies wird durch die Verwendung der erfindungsgemäßen Anlage zur Rechtspolarisation von Quell-, Grund- und Leitungswasser gelöst. Die erfindungsgemäße Anlage wird in einem Abstand von Wasserinstallationsanlagen jeder Art, wie z.B. Rohre, Zuleitungen, Boiler, Wasserspeicher, der die Reichweite von 30 Metern nicht übersteigt, im Boden, z.B. im Fundament, in der Erde, od. dgl. angeordnet, und verhindert dadurch die Ablagerung von Kalk in diesen Einrichtungen, sodaß die Wassergüte wesentlich verbessert wird und in die Nähe der Qualität von Heilwässern kommt.

Nachfolgend wird die Erfindung anhand der in den Zeichnungen dargestellten Ausführungsbeispiele eingehend erläutert. Es zeigt dabei

Fig.1 ein Schaltbild einer Ausführungsform der erfindungsgemäßen Anlage und

Fig.2 einen schematischen Querschnitt der erfindungsgemäßen Anlage in eingebautem Zustand gemäß einer weiteren Ausführungsfom.

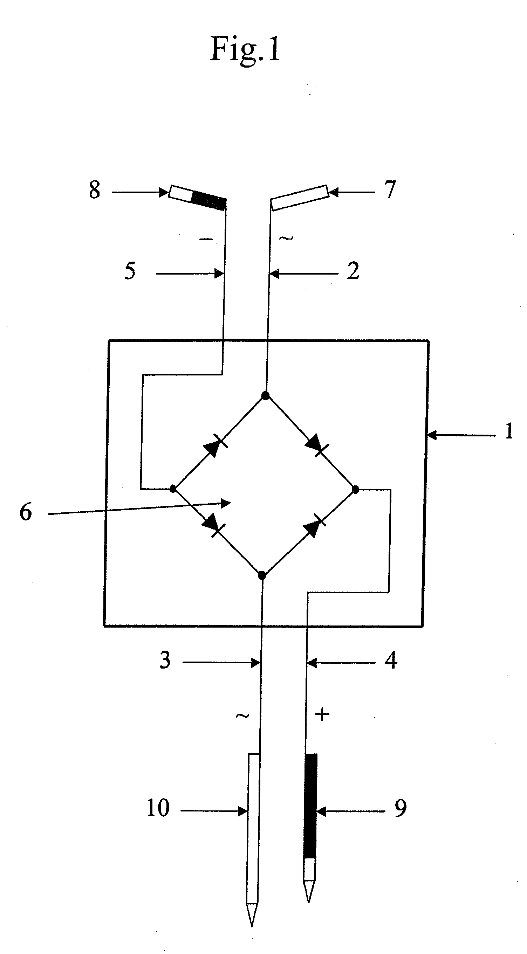

Fig.1 zeigt die der erfindungsgemäßen Anlage zum Entstören von geopathologischen Zonen zugrundeliegende Schaltungsanordnung. Erfindungsgemäß ist eine erste und eine zweite Boden-Einbringungselektrode 7, 10, eine erste und eine zweite Boden-Ausgangselektrode 8, 9 sowie eine Gleichrichterschaltung 1 mit zwei Wechselstrom-Eingängen 2, 3 und zwei Gleichstrom-Ausgängen 4, 5, von denen einer positiv und der andere negativ ist, vorgesehen. Die zwei Boden-Einbringungselektroden 7, 10 sind mit den zwei Wechselstrom-Eingängen 2, 3 und die zwei Boden-Ausgangselektroden 8, 9 mit den zwei Gleichstrom-Ausgängen 4, 5 verbunden.

Um magnetfeldaufbauende Materialien zu vermeiden, sind die beiden Boden-Einbringungselektroden 7, 10 und die beiden Boden-Ausgangselektroden 8, 9 aus einem nicht-magnetischen Material, insbesondere Aluminium, gebildet, ohne auf dieses Material beschränkt zu sein.

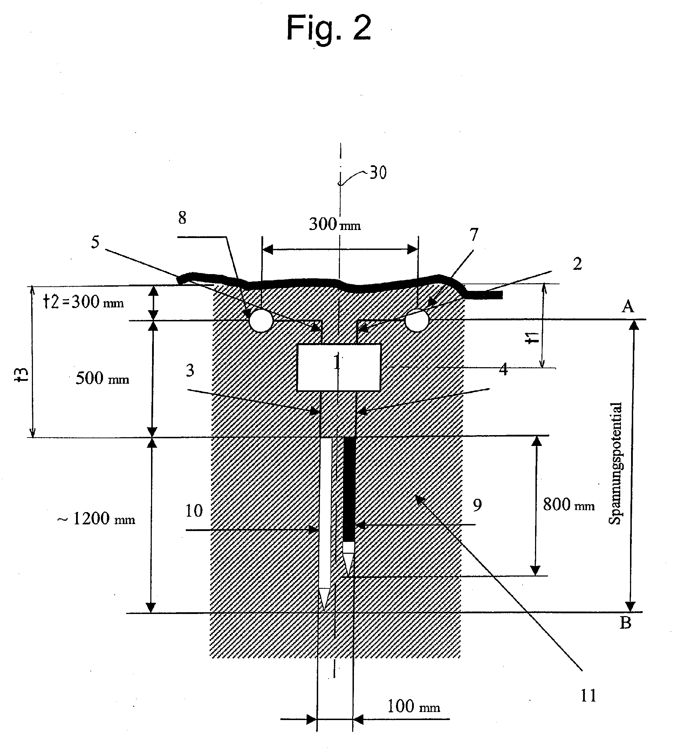

Fig.2 zeigt die im Erdreich 11 eingesetzte erfindungsgemäße Anlage im betriebsbereiten Zustand. Diese kann aber auch in einem Mauerwerk, in einem Fundament oder in einem ähnlichen Bodenbereich untergebracht werden, um geopathologische Interferenzen zu entstören bzw. um die Rechtspolarisation von linksgedrehtem Quellund Leitungswasser hervorzurufen. Der Wirkungsbereich liegt in etwa in einem Umkreis von dreißig Metern.

Dabei ist die Gleichrichterschaltung 1 im Erdreich 11 in einer ersten Tiefe t1 eingebracht, während die erste Boden-Einbringungselektrode 7 und die erste Boden-Ausgangselektrode 8 oberhalb davon in einer zweiten Tiefe t2, die geringer als die erste Tiefe t1 ist, angeordnet sind. Die zweite Tiefe t2 beträgt im gezeigten Ausführungsbeispiel 300 mm.

Unterhalb der Gleichrichterschaltung 1 ist die zweite Boden-Einbringungselektrode 10 und die zweite Boden-Ausgangselektrode 9 in einer dritten Tiefe t3, die größer als die erste Tiefe t1 ist, im Boden eingebracht.

Über die erste und zweite Boden-Einbringungselektrode 7, 10 wird der Gleichrichterschaltung 1, die im Ausführungsbeispiel gemäß Fig.1 als Brückengleichrichter 6 mit vier Halbleiter-Dioden ausgeführt ist, die im Erdreich 11 an bestimmten Punkten vorherrschende Mischspannung zugeführt und von dieser gleichgerichtet. Dabei ist die Gleichrichterschaltung 1 in einem elektrisch isolierenden Kunststoff-Gehäuse eingekapselt, um Erdschlüsse zu vermeiden.

Die von den Boden-Ausgangselektroden 8, 9 negativ und positiv abgegebene Gleichspannung schafft ein neues, gleichgerichtetes Spannungspotential A-B im Erdreich 11, welches bereits nach 0,5 bis 2 Stunden die pathogenen Störzonen beseitigt und somit entstört, wodurch negative Belastungen in positive Energien umgewandelt werden. Da die im Erdreich 11 vorkommende Mischspannung durch natürliche Selbstregeneration unerschöpflich ist, wird somit über die erfindungsgemäße Anlage auch permanent ein gleichgerichtetes Spannungspotential A-B in das Erdreich 11 eingebracht und der ständige Energiefluß über die Boden-Einbringungselektroden 7, 10 zu den Boden-Ausgangselektroden 8, 9 gesichert, die teilweise mit einer elektrisch isolierenden Hülle umgeben sind.

Nach Inbetriebnahme der erfindungsgemäßen Anlage erreicht diese innerhalb von 0,5 bis 2 Stunden ihr maximales Leistungspotential, welches durch die Rückführung der Gleichspannung um ca. 85% erhöht wird. Mit dem erhöhten Leistungspotential in Form linearer Gleichspannung werden die zirkular polarisierten, rotierenden, gravitatorischen Wellenkomponenten des Erdreiches in positive Magnetfelder umgewandelt.

Die erste Boden-Einbringungselektrode 7 und die erste Boden-Ausgangselektrode 8 sind in Einbaulage im wesentlichen waagrecht und parallel zueinander angeordnet, wobei der Abstand der ersten Boden-Einbringungselektrode 7 und der ersten Boden-Ausgangselektrode 8 300 mm beträgt.

Demgegenüber sind die zweite Boden-Einbringungselektrode 10 und die zweite Boden-Ausgangselektrode 9 tiefer im Erdreich 11 angeordnet, nämlich in vertikaler Einbaulage und parallel zueinander, wobei die zweite Boden-Ausgangselektrode 9 kürzer als die zweite Boden-Einbringungselektrode 10 ausgeführt ist.

Dabei beträgt die Länge der zweiten Boden-Einbringungselektrode 10 ungefähr 1200 mm und die Länge der zweiten Boden-Ausgangselektrode 9 ungefähr 800 mm, der Abstand zwischen der zweiten Boden-Einbringungselektrode 10 und der zweiten Boden-Ausgangselektrode 9 ist mit 100 mm festgelegt.

Die zweite Boden-Einbringungselektrode 10 und die zweite Boden-Ausgangselektrode 9 weisen an ihren unteren Enden kegelförmige Spitzen auf, wobei die gegenüberliegenden oberen Enden stumpf ausgebildet und miteinander fluchtend angeordnet sind.

Der Normalabstand zwischen den oberen Enden der zweiten Boden-Einbringungselektrode 10 und der zweiten Boden-Ausgangselektrode 9 einerseits und der ersten Boden-Einbringungselektrode 7 und der ersten Boden-Ausgangselektrode 8 andererseits, der sich in Fig.2 aus der Differenz der Tiefen t3 - t2 berechnet, ist wesentlich für die Funktionsfähigkeit der erfindungsgemäßen Anlage und beträgt 500 mm.

In dem in Fig.2 gezeigten Ausführungsbeispiel ist der Einbau der einzelnen Bestandteile der erfindungsgemäßen Anlage weitgehend symmetrisch vorgenommen worden, um eine möglichst gleichmäßige Wirkung in alle Richtungen zu erzielen. Daher sind die zweite Boden-Einbringungselektrode 10 und die zweite Boden-Ausgangselektrode 9 symmetrisch zu der vertikal zwischen der ersten Boden-Einbringungselektrode 8 und der ersten Boden-Ausgangselektrode 7 verlaufenden Mittelebene 30 angeordnet.

Ohne darauf beschränkt zu sein, sind die Verbindungsleitungen zwischen der Gleichrichterschaltung 1 und den Boden-Einbringungselektroden 7, 10 sowie den Boden-Ausgangselektroden 8, 9 durch isolierte Kupferleitungen gebildet, deren Querschnitt je nach Anforderung im Bereich von 0,15 mm² bis 1 mm² beträgt.

Im Rahmen der Erfindung sind zahlreiche Abänderungen möglich. Auch die Anwendungsgebiete für die erfindungsgemäße Anlage sind vielfältig, insbesondere ist sie auf dem Gebiet der Heilkunde der Human- und Veterinärmedizin, wie auch in vielen anderen Heil- und Therapieformen anwendbar. Weiters eignet sich die erfindungsgemäße Anlage dafür, das Pflanzenwachstum zu beschleunigen und gegen Schädlinge und gegen Krankheiten resistente Aufzuchtformen zu betreiben.

## Patentansprüche

1. Anlage zum Entstören von geopathologischen Zonen, **dadurch gekennzeichnet, daß** eine erste und eine zweite Boden-Einbringungselektrode (7, 10), eine erste und eine zweite Boden-Ausgangselektrode (8, 9) und eine Gleichrichterschaltung (1) mit zwei Wechselstrom-Eingängen (2, 3) und zwei Gleichstrom-Ausgängen (4, 5), von denen einer positiv und der andere negativ ist, vorgesehen sind, wobei die zwei Boden-Einbringungselektroden (7, 10) mit den zwei Wechselstrom-Eingängen (2, 3) und die zwei Boden-Ausgangselektroden (8, 9) mit den zwei Gleichstrom-Ausgängen (4, 5) verbunden sind, und daß im Betrieb die Gleichrichterschaltung (1) in einer ersten Tiefe (t1), die erste Boden-Einbringungselektrode (7) und die erste Boden-Ausgangselektrode (8) in einer zweiten Tiefe (t2), die geringer als die erste Tiefe (t1) ist, sowie die zweite Boden-Einbringungselektrode (10) und die zweite Boden-Ausgangselektrode (9) in einer dritten Tiefe (t3), die größer als die erste Tiefe (t1) ist, im Boden eingebracht sind.

2. Anlage nach Anspruch 1, **dadurch gekennzeichnet, daß** der Boden durch Erdreich (11), ein Mauerwerk oder ein Fundament gebildet ist.

3. Anlage nach Anspruch 1 oder 2, **dadurch gekennzeichnet, daß** die Gleichrichterschaltung (1) durch einen Brückengleichrichter (6) gebildet ist.

4. Anlage nach Anspruch 1, 2 oder 3, **dadurch gekennzeichnet, daß** die Gleichrichterschaltung (1) in einem elektrisch isolierenden Kunststoff-Gehäuse eingekapselt ist.

5. Anlage nach einem der Ansprüche 1 bis 4, **dadurch gekennzeichnet, daß** die Boden-Ausgangselektroden (8, 9) teilweise mit einer elektrisch isolierenden Hülle umgeben sind.

6. Anlage nach einem der Ansprüche 1 bis 5, **dadurch gekennzeichnet, daß** die erste Boden-Einbringungselektrode (7) und die erste Boden-Ausgangselektrode (8) in Einbaulage im wesentlichen waagrecht und parallel zueinander angeordnet sind.

7. Anlage nach Anspruch 6, **dadurch gekennzeichnet, daß** der Abstand der ersten Boden-Einbringungselektrode (7) und der ersten Boden-Ausgangselektrode (8) 300mm beträgt.

8. Anlage nach einem der vorhergehenden Ansprüche, **dadurch gekennzeichnet, daß** die zweite Boden-Einbringungselektrode (10) und die zweite Boden-Ausgangselektrode (9) in Einbaulage vertikal und parallel zueinander angeordnet sind.

9. Anlage nach einem der vorhergehenden Ansprüche, **dadurch gekennzeichnet, daß** die Länge der zweiten Boden-Einbringungselektrode (10) ungefähr 1200 mm und die Länge der zweiten Boden-Ausgangselektrode (9) ungefähr 800 mm beträgt, und daß der Abstand zwischen der zweiten Boden-Einbringungselektrode (10) und der zweiten Boden-Ausgangselektrode (9) 100 mm beträgt.

10. Anlage nach Anspruch 8 oder 9, **dadurch gekennzeichnet, daß** die zweite Boden-Einbringungselektrode (10) und die zweite Boden-Ausgangselektrode (9) an ihren unteren Enden kegelförmige Spitzen aufweisen, und daß die gegenüberliegenden oberen Enden stumpf ausgebildet und miteinander fluchtend angeordnet sind.

11. Anlage einem der Ansprüche 6 bis 10, **dadurch gekennzeichnet, daß** der Normalabstand (t3-t2) zwischen den oberen Enden der zweiten Boden-Einbringungselektrode (10) und der zweiten Boden-Ausgangselektrode (9) einerseits und der ersten Boden-Einbringungselektrode (7) und der ersten Boden-Ausgangselektrode (8) andererseits 500 mm beträgt.

12. Anlage nach einem der vorhergehenden Ansprüche, **dadurch gekennzeichnet, daß** die zweite Tiefe (t2) 300 mm beträgt.

13. Anlage nach einem der Ansprüche 6 bis 12, **dadurch gekennzeichnet, daß** die zweite Boden-Einbringungselektrode (10) und die zweite Boden-Ausgangselektrode (9) symmetrisch zu der vertikal zwischen der ersten Boden-Einbringungselektrode (8) und der ersten Boden-Ausgangselektrode (7) verlaufenden Mittelebene (30) angeordnet sind.

14. Anlage nach einem der vorhergehenden Ansprüchen, **dadurch gekennzeichnet, daß** die beiden Boden-Einbringungselektroden (7, 10) und die beiden Boden-Ausgangselektroden (8, 9) aus einem nicht-magnetischen Material, insbesondere Aluminium, gebildet sind.

15. Anlage nach einem der vorhergehenden Ansprüche, **dadurch gekennzeichnet, daß** die Verbindungsleitungen zwischen der Gleichrichterschaltung (1) und den Boden-Einbringungselektroden (7, 10) sowie den Boden-Ausgangselektroden (8, 9) durch isolierte Kupferleitungen gebildet sind, deren Querschnitt im Bereich von 0,15 mm² bis 1 mm² beträgt.

16. Verwendung der Anlage nach einem der Ansprüche 1 bis 15 zur Rechtspolarisation von Quell-, Grund- und Leitungswasser.
